(19) Europäisches Patentamt
European Patent Office
Office européen des brevets

(11) Veröffentlichungsnummer: **0 022 074**
**A2**

# (12) EUROPÄISCHE PATENTANMELDUNG

(21) Anmeldenummer: **80810196.8**

(22) Anmeldetag: **12.06.80**

(51) Int. Cl.$^3$: **C 07 C 161/00**

(30) Priorität: **18.06.79 CH 5669 79**

(43) Veröffentlichungstag der Anmeldung:
**07.01.81 Patentblatt 81/1**

(84) Benannte Vertragsstaaten:
**AT BE CH DE FR IT LI LU NL SE**

(71) Anmelder: **CIBA-GEIGY AG**
**Patentabteilung Postfach**
**CH-4002 Basel(CH)**

(72) Erfinder: **Ledouble, Jean-Pierre**
**Rue des Landes 10**
**F-68300 Rosenau(FR)**

(72) Erfinder: **Müller, Klaus, Dr.**
**Steinrebenstrasse 76**
**F-4153 Reinach(CH)**

(54) **Verfahren zur Herstellung von Bis-(0-(1-Alkylthioäthylimino)-N-methylcarbaminsäure)-N,N'-sulfiden.**

(57) Es wird ein Verfahren zur Herstellung von Bis-[O-(l-Alkylthioäthylimino)-N-methylcarbaminsäure]-N, N'-sulfiden der Formel

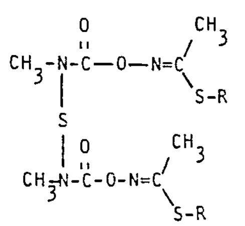

in welcher R eine geradkettige oder verzweigte Alkylgruppe mit 1 bis 5 Kohlenstoffatomen bedeutet, durch Umsetzung eines O-(l-Alkylthioäthylimino)- N-methylcarbamats der Formel

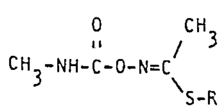

in Gegenwart einer Base mit Schwefeldichlorid bzw. Dischwefeldichlorid in einem Lösungsmittel mit einer Dielektrizitätskonstanten von 2,0 bis 2,5 und einem Dipolmoment von 0 bis 1 beschrieben.

Die Bis-[O-(1-alkylthioäthylimino)-N-methylcarbaminsäure]-N,N'-sulfide der obigen Formel besitzen insektizide Wirkung und eignen sich insbesondere zur Bekämpfung von Schädlingen von Baumwollpflanzen und Schmeissfliegen.

EP 0 022 074 A2

Croydon Printing Company Ltd.

5-12394/=

Verfahren zur Herstellung von Bis-[O-(1- Alkylthioäthylimino)-N-methylcarbaminsäure]-N,N'-sulfiden.

Die vorliegende Erfindung betrifft ein Verfahren zur Herstellung von Bis-[O-(1-Alkylthioäthylimino)-N-methylcarbaminsäure]-N,N'-sulfiden der Formel

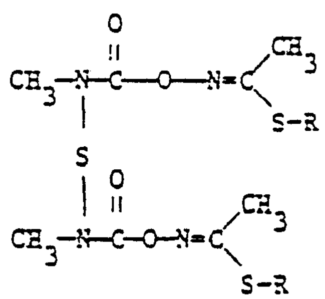

(I)

in welcher R eine geradkettige oder verzweigte Alkylgruppe mit 1 bis 5 Kohlenstoffatomen bedeutet, durch Umsetzung eines O-(1-Alkylthioäthylimino)-N-methylcarbamats der Formel

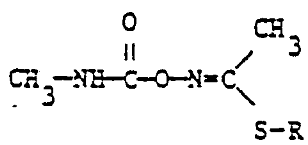

(II)

in welcher R die oben angegebene Bedeutung hat, in Gegenwart einer Base mit Schwefeldichlorid bzw. Dischwefeldichlorid.

Die Bis-[O-(1-Alkylthioäthylimino)-N-methylcarbaminsäure]-N,N'-sulfide der Formel I besitzen insektizide Wirkung und eignen sich insbesondere zur Bekämpfung von Schädlingen von Baumwollpflanzen und Schmeissfliegen (Lucilia sericata).

BAD ORIGINAL

Es ist bereits aus der Deutschen Offenlegungsschrift 2,530,439 bekannt, die Bis-[O-(1-Alkylthioäthylimino)-N-methylcarbaminsäure]-N,N'-sulfide der Formel I durch Umsetzung eines O(1-Alkylthioäthylimino)-N-methylcarbamat der Formel II in einem inerten Lösungsmittel und in Gegenwart einer Base bei Temperaturen von -10 bis 100° C mit Schwefeldichlorid bzw. Dischwefeldichlorid herzustellen. Dabei werden als inerte Lösungsmittel Aether, aliphatische und aromatische Kohlenwasserstoffe und Ketone und als Basen insbesondere tertiäre Amine, wie Trialkylamine, Pyridine und N,N-Dialkylaniline genannt. Diese Methode ist insofern nachteilig, als die Reaktion bei Verwendung der genannten Lösungsmittel uneinheitlich verläuft, was zu einer Verminderung der Ausbeute und der Produkte-Qualität führt. Ausserdem werden lange Reaktionszeiten benötigt.

Es wurde nun gefunden, dass man die vorgenannten Nachteile vermeiden kann, wenn man die Umsetzung des O-(1-Alkylthioäthylimino)-N-methylcarbamats der Formel II mit Schwefeldichlorid bzw. Dischwefeldichlorid in Gegenwart einer Base in einem Lösungsmittel mit einer Dielektrizitätskonstanten von 2,0 bis 2,5 und einem Dipolmoment von 0 bis 1 durchführt.

Als Lösungsmittel können erfindungsgemäss reine unpolare Lösungsmittel oder Mischungen von unpolaren Lösungsmitteln mit polaren Lösungsmitteln mit den vorgenannten Eigenschaften verwendet werden. Geeignete unpolare Lösungsmittel sind halogenierte Kohlenwasserstoffe, wie Kohlenstofftetrachlorid, 1,1,2-Trifluor-1,2,2-trichloräthan (Flugen® 113) und Tetrachloräthylen. Geeignete Mischungen von unpolaren und polaren Lösungsmitteln sind Mischungen von Kohlenstofftetrachlorid mit Chloroform oder 1,2-Dichloräthan, Mischungen von Hexan mit Methylenchlorid oder 1,2-Dichloräthan sowie Mischungen von Benzol- oder Cyclohexan mit 1,2-Dichloräthan.

Bei den vorgenannten Mischungen von unpolaren und polaren Lösungsmitteln ist der Anteil an polarem Lösungsmittel jeweils so zu bemessen, dass für die Mischung eine Dielektrizitätskonstante von

BAD ORIGINAL

2,0 bis 2,5 und ein Dipolmoment von 0 bis 1 resultiert. Um dies zu erreichen, beträgt der Anteil an polarem Lösungsmittel je nach dessen Polarität zwischen 2 und 20 Vol.%.

Besonders gute Resultate werden erhalten, wenn man 1,1,2-Trifluor-1,2,2-trichloräthan oder Tetrachloräthylen als Lösungsmittel verwendet. Die Verwendung von 1,1,2-Trifluor-1,2,2-trichloräthan und Tetrachloräthylen als Lösungsmittel ist daher bevorzugt. Besonders bevorzugt ist die Verwendung von 1,1,2-Trifluor-1,2,2-trichloräthan als Lösungsmittel.

Die erfindungsgemässen Lösungsmittel werden in Mengen von 500 bis 1000 ml, vorzugsweise 500 bis 700 ml pro Mol O-(1-Alkylthioäthylimino)-N-methylcarbamat der Formel II verwendet.

Als geeignete Basen, in deren Gegenwart die Umsetzung von O-(1-Alkylthioäthylimino)-N-methylcarbamaten der Formel II mit Schwefeldichlorid bzw. Dischwefeldichlorid durchgeführt werden kann, kommen Pyridin, Alkylpyridine, wie Picolin und Lutidin, und in p-Stellung substituierte N,N-Dialkylaniline in Betracht, wobei der p-Substituent in den N,N-Dialkylanilinen, Halogen, Alkyl oder Alkoxy sein kann. Vorzugsweise wird Pyridin als Base verwendet.

Die vorgenannten Basen können in stöchiometrischer Menge oder in einem Ueberschuss von bis zu 100 % verwendet werden. Vorzugsweise werden die Basen in einem Ueberschuss von 50 % bezogen auf die stöchiometrische Menge verwendet.

Die erfindungsgemässe Umsetzung eines O-(1-Alkylthioäthylimino)-N-methylcarbamats der Formel II mit Schwefeldichlorid bzw. Dischwefeldichlorid wird bei Temperaturen von -10° C bis 50° C, vorzugsweise 0 bis +30° C durchgeführt und nimmt 2 bis 10 Stunden in vielen Fällen nur 4 bis 6 Stunden in Anspruch.

BAD ORIGINAL

Gemäss einer bevorzugten Ausführungsform wird das erfindungsgemässe Verfahren in der Weise durchgeführt, dass man Schwefeldichlorid bzw. Dischwefeldichlorid und das Lösungsmittel, vorzugsweise 1,1,2-Trifluor-1,2,2-trichloräthan oder Tetrachloräthylen, vorlegt, dann zunächst unter Kühlung die Base, vorzugsweise Pyridin, und schliesslich das O-(1-Alkylthioäthylimino)-N-methylcarbamat der Formel II zugibt und das Reaktionsgemisch während 4 bis 6 Stunden bei einer Temperatur von 0 bis +30° C hält.

Die als Ausgangsmaterialien zu verwendenden O-(1-Alkylthioäthylimino)-N-methylcarbamate der Formel II sind aus der britischen Patentschrift 1,138,347 bekannt. Sie können beispielsweise durch Umsetzung von Acetaldehyd mit Hydroxylamin zu Acetaldoxim, weitere Umsetzung des durch Chlorierung von Acetaldoxim erhaltenen Acethydroxamsäurechlorids mit Alkalialkylmercaptid zum entsprechenden 1-Alkylthioacetaldoxim und dessen weitere Umsetzung mit Methylisocyanat hergestellt werden.

Mit dem erfindungsgemässen Verfahren wird es möglich, die Bis-[O-(1-Alkylthioäthylimino)-N-methylcarbaminsäure]-N,N'-sulfide der Formel I unter weitgehender Vermeidung der Bildung unerwünschter Nebenprodukte in Ausbeuten zwischen 80 und 85 % der Theorie herzustellen. Die Endprodukte werden in einer Reinheit von 95 % erhalten und können ohne weitere Reinigung verwendet werden. Ausserdem ermöglicht die Verwendung der erfindungsgemäss vorgeschlagenen Lösungsmittels eine Verkürzung der Reaktionszeit auf weniger als 10 Stunden. Das erfindungsgemässe Verfahren eignet sich daher sehr gut für die Herstellung der insektiziden Wirkstoffe der Formel I in technischem Massstab.

Das erfindungsgemässe Verfahren wird durch die folgenden Beispiele näher erläutert:

BAD ORIGINAL

Beispiel 1: In 285 ml 1,1,2-Trifluor-1,2,2-trichloräthan (Flugen® 113) werden 31,0 g (0,30 Mol) Schwefeldichlorid eingetragen. In die erhaltene Mischung werden bei 5 bis 10° C unter Rühren 55,0 g Pyridin eingetropft. Nach beendigter Zugabe des Pyridins werden 75,0 g (0,46 Mol) O-(1-Methylthioäthylimino)-N-methylcarbamat bei 10° C zugegeben. Anschliessend wird das Reaktionsgemisch auf 30° C erwärmt und 4 Stunden bei dieser Temperatur gerührt. Dann wird bei Raumtemperatur filtriert, der Filterrückstand mit Wasser verrührt, nochmals filtriert und bei 40° C im Vakuum getrocknet. Es werden 69,0 g Bis-[O-(1-methylthioäthylimino)-N-methylcarbaminsäure]-N,N'-sulfid (95%ig) erhalten, was einer Ausbeute von 80 % der Theorie, bezogen auf O-(1-Methylthioäthylimino)-N-methylcarbamat, entspricht.

Beispiel 2: Nach der in Beispiel 1 angegebenen Methode werden 31,0 g (0,3 Mol) Schwefeldichlorid und 75 g (0,46 Mol) O-(1-Methylthioäthylimino)-N-methylcarbamat in 325 ml Tetrachloräthylen umgesetzt, wobei das Reaktionsgemisch nach Zugabe des O-(1-Methyltioäthylimino)-N-methylcarbamat 5 Stunden bei 30° C gerührt wird. Auf diese Weise werden ebenfalls 69,0 g 95%iges Bis-[O-(Methylthioäthylimino)-N-methylcarbaminsäure]-N,N'-sulfid (80 % der Theorie) erhalten.

BAD ORIGINAL

Patentansprüche

1. Verfahren zur Herstellung von Bis-[O-(1-Alkylthioäthylimino)-N-methylcarbaminsäure]-N,N'-sulfiden der Formel I

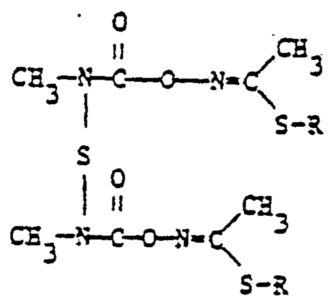

(I)

in welcher R eine geradkettige oder verzweigte Alkylgruppe mit 1 bis 5 Kohlenstoffatomen bedeutet, durch Umsetzung eines O-(1-Alkylthioäthylimino)-N-methylcarbamats der Formel

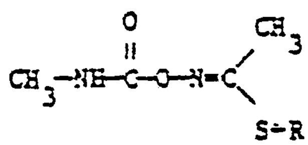

(II)

in welcher R die oben angegebene Bedeutung hat, in Gegenwart einer Base mit Schwefeldichlorid bzw. Dischwefeldichlorid in einem inerten Lösungsmittel, dadurch gekennzeichnet, dass man ein Lösungsmittel mit einer Dielektrizitätskonstanten von 2,0 bis 2,5 und einem Dipolmoment von 0 bis 1 verwendet.

2. Verfahren nach Anspruch 1, dadurch gekennzeichnet, dass man als Lösungsmittel einen halogenierten Kohlenwasserstoff verwendet.

3. Verfahren nach Anspruch 1, dadurch gekennzeichnet, dass man als Lösungsmittel Kohlenstofftetrachlorid, 1,1,2-Trifluor-1,2,2-trichloräthan oder Tetrachloräthylen verwendet.

4. Verfahren nach Anspruch 1, dadurch gekennzeichnet, dass man als Lösungsmittel 1,1,2-Trifluor-1,2,2-trichloräthan oder Tetrachloräthylen verwendet.

BAD ORIGINAL

5. Verfahren nach Anspruch 1, dadurch gekennzeichnet, dass man als Lösungsmittel 1,1,2-Trifluor-1,2,2-trichloräthan verwendet.

6. Verfahren nach Anspruch 1, dadurch gekennzeichnet, dass man das Lösungsmittel in Mengen von 500 bis 1000 ml pro Mol O-(1-Alkylthioäthylimino)-N-methylcarbamat der Formel II verwendet.

7. Verfahren nach Anspruch 1, dadurch gekennzeichnet, dass man das Lösungsmittel in Mengen von 500 bis 700 ml pro Mol O-(1-Alkylthioäthylimino)-N-methylcarbamat der Formel II verwendet.

8. Verfahren nach Anspruch 1, dadurch gekennzeichnet, dass man die Umsetzung eines O-(1-Alkylthioäthylimino)-N-methylcarbamats der Formel II mit Schwefeldichlorid bzw. Dischwefeldichlorid in Gegenwart von Pyridin durchführt.

9. Verfahren nach Anspruch 1, dadurch gekennzeichnet, dass man die Umsetzung O-(1-Alkylthioäthylimino)-N-methylcarbamts der Formel II mit Schwefeldichlorid bzw. Dischwefeldichlorid bei Temperaturen von -10° C bis 50° C, vorzugsweise 0° C bis 30° C durchführt.

10. Verfahren nach Anspruch 1, dadurch gekennzeichnet, dass man die Umsetzung eines O-(1-Alkylthioäthylimino)-N-methylcarbamats der Formel II mit Schwefeldichlorid bzw. Dischwefeldichlorid in der Weise durchführt, dass man das Schwefeldichlorid bzw. Dischwefeldichlorid und das Lösungsmittel vorlegt, dann zunächst unter Kühlung die Base und schliesslich das O-(1-Alkylthioäthylimino)-N-methylcarbamat der Formel II zugibt, und das Reaktionsgemisch während 4 bis 6 Stunden bei einer Temperatur von 0 bis +30° C hält.

BAD ORIGINAL